# DEMANDE DE BREVET EUROPEEN

(11) **EP 1 266 578 A1**
(43) Date de publication de la demande: **18.12.2002**
(21) Numéro de dépôt: 02356102.0
(22) Date de dépôt: 06.06.2002
(51) Int. Cl.: A23K 1/16, A23K 1/18, A61K 31/11, A61K 35/78

(54) **Préparation destinée a l'animal, en particulier la volaille**

(30) Priorité: 14.06.2001 FR 0107784
(71) Demandeur: Phytosynthese, 03800 Gannat (FR)
(72) Inventeur: Greuter, Amaury, 49380 Thouarcé (FR); Picaud, Thierry, 63440 Saint Gal sur Sioule (FR)
(74) Mandataire: Denjean, Eric

(57) **Abrégé**

Utilisation d'une préparation contenant du cinnamaldéhyde pour la fabrication d'une composition destinée au traitement préventif ou curatif des pathologies animales au cours desquelles une présence surnuméraire de germes anaérobies est observée.

## Description

L'invention concerne une préparation destinée à l'animal, en particulier la volaille. Elle se rapporte également à l'utilisation de ladite préparation pour la fabrication d'une composition destinée au traitement préventif ou curatif des pathologies animales, au cours desquelles une présence surnuméraire des germes anaérobie, est observée.

Dans la suite de la description, par l'expression "anaérobie", on désigne des germes évoluant en abscence totale (anaérobie stricte) ou quasi-totale (microaérophilie) d'oxygène. L'invention est plus particulièrement décrite en relation avec les bactéries du genre Clostridium (anaérobie stricte), en particulier Clostridium perfringens et du genre Brachyspira (microaérophile), en particulier Brachyspira hyodysenteriae.

Les germes Clostridium perfringens sont bien connus pour leur implication dans les troubles digestifs, non seulement chez l'homme, mais également chez l'animal terrestre non humain. Chez ce dernier, exclusivement concerné par la préparation objet de l'invention, les germes se manifestent dans différentes pathologies.

Parmi les animaux susceptibles d'être infectés par Clostridium perfringens, on distingue tout d'abord les volailles et en particulier les dindes, poulets, pintades, gibier à plume, perdrix, faisans mais également les porcs, les ruminants (bovins, ovins et caprins) et les lapins sans pour autant que cette liste soit limitative. Ces animaux développent des pathologies en liaison avec les germes précités.

Chez les volailles tout d'abord, on connait la dysbactériose, qui se manifeste par des troubles digestifs mineurs dus à un déséquilibre de la flore bactérienne, en raison d'une quantité anormale de Clostridium perfringens. Par quantité anormale, on entend tout écart significatif par rapport à une situation normale, qui peut se résumer aux chiffres suivants :
Germes totaux d'un poulet adulte : 10 ^{10 à 12} germe/gr de matière fécale, dont :
   - 99 % environ de flore non pathogène : lactobacillus, bifidobactérium, bactéroïdes ;
   - 1 % environ de Echerichia coli, staphylococcus, enterococcus ;
   - moins de 1 % d'autres germes dont Clostridium perfringens.

Le milieu vétérinaire considère qu'une présence supérieure à 10⁴ de ces germes/gr est un signe de dysbactériose.

Parmi les différentes pathologies concernées plus particulièrement par l'invention, on connaît également les entérites non spécifiques se manifestant par des diarrhées et les entérites nécrotiques, lesquelles se caractérisent par une inflammation importante du tractus digestif, pouvant conduire jusqu'à la mort de l'animal. Parmi les entérites, on connaît enfin l'entérite frilositée se développant plus particulièrement chez la pintade, qui se caractérise par une présence de diarrhée et d'une modification du comportement des pintades, qui adoptent une attitude prostrée.

Les porcs et les ruminants (bovins, ovins et caprins) peuvent être également infectés par les germes du genre Clostridium. On parle dans ce cas d'entérotoxémie. L'entérotoxémie est une pathologie connue pour son évolution fulgurante. La cause principale est un stress quelconque intervenant sur un régime alimentaire très riche, qui favorise un déséquilibre de flore. Les germes se développent à très grande vitesse. La production de toxines peut tuer l'animal avant l'apparition de symptômes marqués (diarrhée).

On connaît enfin l'entérocolite du lapin, dont on suppose que l'origine est reliée à la présence d'un virus. Les élevages sont touchés par des mortalités de 15 à 50 % et plus. En déprimant l'immunité, le virus facilite l'infection du lapin par des germes opportunistes du type Clostridium ou Coli, lesquels induisent la mortalité.

Un certain nombre de traitements ont été proposés pour éradiquer le Clostridium perfringens impliqué dans les pathologies se développant chez l'animal. Un premier traitement consiste à administrer à l'animal un antibiotique choisi parmi notamment l'amoxicilline, l'érythromycine ou encore la lincomycine. Si ces antibiotiques permettent d'obtenir de bons résultats 24 heures seulement après la première administration, ils présentent cependant l'inconvénient de ne pas être spécifiques et de conduire outre à la destruction recherchée du Clostridium perfringens, à celle du reste de la flore digestive. Le spectre d'activité large des antibiotiques fragilise donc la flore digestive, rendant ainsi les animaux fragiles et candidats aux rechutes. En outre, on observe de plus en plus des phénomènes de résistance de ce germe, aux antibiotiques.

Un autre traitement décrit dans le document WO 99/59430 consiste à administrer des substances naturelles à l'animal, incorporées directement dans la nourriture ou dans la boisson. Plus précisément, ce document décrit l'utilisation d'une composition à base de thymol pour la fabrication d'un additif alimentaire destiné au traitement curatif ou préventif des pathologies dans lesquelles est impliqué le Clostridium perfringens.

Le document WO 00/47069 décrit une composition destinée au traitement des diarrhées sécrétrices de l'humain ou de l'animal due à la présence de Clostridium perfringens contenant un extrait de latex riche en proanthocyanidine avec des plantes, en particulier de la cannelle. Ce document indique clairement que l'activité bactéricide vis-à-vis du Clostridium est due au polymère de proanthocyanidine, la cannelle étant uniquement mentionnée pour son action dans les désordres gastro-intestinaux. En particulier, il est indiqué que la cannelle a une action vis-à-vis du germe Hélicobacter pylori, connu pour évoluer en milieu aérobie.

Classiquement, il est admis que Brachyspira hyodysenteriae a un spectre d'hôtes étroit, limité au porc, même si cette bactérie est capable d'infecter de manière transitoire et sans symptôme d'autres espèces animales en contact avec les fèces de porcs infectés : rats, souris, chiens, étourneaux. Plus récemment, l'isolement de souches de Brachyspira hyodysenteriae dans des élevages de rhéas (Rhea americana) dont les animaux présentaient des lésions de typhlocolite nécrosante suggère un spectre d'hôtes plus large. Chez le rhéa, les animaux présentant des signes cliniques sont âgés de moins de 6 mois. Les principaux signes cliniques consistent en une perte de poids et une diarrhée aqueuse mais, le plus souvent, les animaux meurent sans symptôme préalable et le taux de mortalité peut atteindre 25 à 80 %. A l'autopsie, les caecums sont dilatés, le contenu caecal contient des pseudomembranes et du mucus et la muqueuse caecale est épaissie et présente des ulcères. Les souches isolées de rhéas sont aptes à reproduire la maladie chez des poulets et des dindes âgées de 1 jour mais les signes cliniques sont moins sévères.

La souris joue en rôle important dans la contamination des porcs car elle peut se contaminer avec un faible nombre de bactéries (102 unités formant colonies) et elle peut excréter le germe dans ses fèces durant 6 mois. Les autres espèces animales restent porteuses durant un temps plus bref : 13 jours pour le chien, 2 jours pour le rat, 8 heures pour l'étoumeau. Les mouches peuvent disséminer des germes viables durant au moins 4 heures. La survie du germe dans le milieu environnant est parfois considérable : 48 jours dans des fèces de porcs maintenues à une température comprise entre 0 et 10°C, 61 jours dans des fèces diluées au 1/10 dans de l'eau et stockées à 5°C. En revanche, à des températures plus élevées, le temps de survie dans les fèces diminue considérablement : 7 jours à 25°C et 24 heures à 37°C.

Brachyspira hyodysenteriae est l'agent de la dysenterie porcine (appelée également diarrhée hémorragique ou entérite hémorragique), maladie identifiée dans tous les pays où l'élevage porcin est développé et affectant principalement les porcs en engraissement (mais les truies et les porcelets sevrés peuvent également présenter des signes cliniques). En France, la prévalence de la dysenterie a atteint 17 % en 1969 puis, depuis l'utilisation du carbadox, elle a diminué aux alentours de 8 à 9 %. La dysenterie porcine est une maladie dont les répercussions économiques sont importantes du fait de la mortalité et, surtout, du coût des traitements et des retards de croissance engendrés. A titre d'exemple, le coût de l'infection peut représenter jusqu'à 19 $ par porc en Grande-Bretagne, l'âge d'abattage des animaux peut être retardé de 28 jours, le coût annuel de la maladie aux U.S.A. est de l'ordre de 115,2 millions de $.

Le principal mode de contamination d'un élevage résulte de l'introduction d'animaux infectés. Les animaux guéris représentent un danger important car ils peuvent excréter le germe pendant plus de 70 jours. Toutefois, généralement, cette excrétion est plus brève : environ 50 % des animaux convalescents restent excréteurs au-delà de 10 jours et seuls 21 % excrètent le germe au-delà de 18 jours. D'autres modes de contamination ont été décrits : contamination par des effluents, contamination par des animaux (principalement souris mais aussi chiens et mouches), nourriture contaminée, objets souillés (notamment des bottes),... Les rongeurs, les chiens et dans une moindre mesure les mouches sont incriminées dans le maintien de l'infection après le départ des porcs pour l'abattoir.

Expérimentalement, la dose infectante pour le porc est de l'ordre de 105 unités formant colonies. La période d'incubation varie de 5 à 37 jours avec une moyenne de 11 jours mais, durant cette période d'incubation, les animaux peuvent déjà être excréteurs car ils éliminent la bactérie entre 3 et 18 jours après un contact infectant.

Le problème que se propose de résoudre l'invention est de proposer une nouvelle composition contenant au moins une substance naturelle susceptible d'inhiber le développement des germes anaérobies, en particulier ceux du genre Clostridium ou Brachyspira, chez l'animal. Un autre objectif de l'invention est de proposer un produit qui soit efficaces dans des quantités les plus faibles possible.

Or, le Demandeur a constaté que de manière tout a fait inattendue, le cinnamaldéhyde inhibait le développement, chez l'animal des germes appartenant à la catégorie précitée (anaérobie), et notamment ceux du genre Clostridium, en particulier Clostridium perfringens et ceux du genre Brachyspira, en particulier Brachyspira hyodysenteriae.

L'invention a donc pour objet l'utilisation d'une préparation contenant du cinnamaldéhyde pour la fabrication d'une composition destinée au traitement curatif ou préventif des pathologies animales au cours desquelles une présence surnuméraire de germes anaérobies, est observée.

Selon un premier mode de réalisation, le germe anaérobie appartient au genre Clostridium, en particulier Clostridium perfringens.

Selon un second mode de réalisation, le germe anaérobie appartient au genre Brachyspira, en particulier Brachyspira hyodysenteriae.

Bien entendu, le cinnamaldéhyde peut être obtenu par synthèse, mais également par extraction végétale, en particulier à partir de cannelle, dont on sait qu'elle contient une telle substances. En d'autres termes et selon une première caractéristique de l'invention, la source de cinnamaldéhyde est un extrait de cannelle. En pratique, la cannelle est de la cannelle de Chine, connue sous la dénomination latine *cinnamomum cassia* ou de la cannelle connue sous la dénomination *cinnamomum ceylani*. Pour obtenir un extrait le plus concentré possible en cinnamaldéhyde, l'extrait utilisé se présente sous la forme d'une huile essentielle.

Ainsi et selon une autre caractéristique, l'huile essentielle de cannelle contient entre 50 et 90 % en poids de cinnamaldéhyde, avantageusement entre 70 et 85 % en poids. Ladite huile essentielle contient en outre du methoxycinnamaldéhyde, en pratique à raison de 1 à 20% en poids.

Bien entendu, l'huile essentielle de cannelle peut être obtenue par toutes les techniques connue de l'homme du métier, en particulier par entraînement à la vapeur.

Pour rendre l'huile essentielle administrable par voie orale, la préparation de l'invention se présente, selon un premier mode de réalisation, sous forme d'une émulsion stabilisée, dans laquelle l'huile essentielle est dispersée dans une phase aqueuse en présence d'un agent dispersant. Préférentiellement, l'agent dispersant est un dispersant alimentaire, notamment choisi parmi les saponines ou les tweens.

En pratique, les tweens, quand ils sont utilisés, représentent entre 20 et 40 % de l'huile essentielle, avantageusement 30 %. S'agissant des saponines, celles-ci représentent en pratique entre 0,5 et 5 %, avantageusement 1,5 % en poids de l'huile essentielle.

Dans un autre mode de réalisation, la préparation se présente sous forme d'une poudre sur laquelle est adsorbée l'huile essentielle. En pratique, la poudre est constituée de silice ou issue de remoulage, en particulier de blé.

Dans un troisième mode de réalisation, l'huile essentielle peut être micro-encapsulée, en particulier par des β-cyclodextrines, procédé conduisant cependant à augmenter de manière importante le coût de la préparation.

Selon une autre caractéristique de l'invention, la préparation est administrée comme déjà dit par voie orale. Elle peut être incorporée soit dans l'alimentation et se présenter sous forme d'un aliment, soit dans l'eau de boisson et se présenter sous forme d'une eau de boisson.

Lorsqu'il s'agit d'une eau de boisson, la préparation est incorporée à raison de 0.1 à 3 ml, avantageusement de 0.35 à 1 ml par litre d'eau de boisson.

Les pathologies et animaux concernés par la préparation de l'invention lorsque le germe du genre Clostridium est en cause comprennent les dysbactérioses, les entérites non spécifiques, les entérites nécrotiques, mais également des entérites frilositées se développant chez les volailles (en particulier, les dindes, poulets, pintades, gibier à plume, perdrix, faisans).

De même, la préparation de l'invention concerne le traitement de la dysenterie porcine provoquée par Brachyspira hyodysenteriae.

Dans toutes ces pathologies, lorsque la préparation est utilisée dans le cadre d'un traitement préventif, la quantité de cinnamaldéhyde administrée est comprise entre 0,5 x 10⁻³ et 50 x 10⁻³ mg/kg de poids vif/jour.

Par exemple, la quantité de cinnamaldéhyde administrée est comprise :
- entre 1 x 10⁻³ et 9 x 10⁻³ mg/kg de poids vif/jour pour le poulet,
- entre 0,6 x 10⁻³ et 17 x 10⁻³ mg/kg de poids vif/jour pour la dinde,
- entre 1,5 x 10⁻³ et 35 x 10⁻³ mg/kg de poids vif/jour pour le canard.

En revanche, lorsqu'il s'agit d'un traitement curatif en particulier au moyen de l'eau de boisson, la quantité de cinnamaldéhyde administrée est comprise entre 3 x 10⁻³ et 350 x 10⁻³ mg/kg de poids vif/jour.

Par exemple, la quantité de cinnamaldéhyde administrée est comprise :
- entre 9 x 10⁻³ mg/kg et 77 x 10⁻³ mg/kg pour le poulet,
- entre 6 x 10⁻³ mg/kg et 147 x 10⁻³ mg/kg pour la dinde,
- entre 15 x 10⁻³ mg/kg et 295 x 10⁻³ mg/kg pour le canard.

Selon une autre caractéristique, la préparation de l'invention peut également être utilisée pour la fabrication d'une composition destinée au traitement préventif des entérotoxémies, lesquelles se développent plus particulièrement chez le porc et les ruminants (ovins, bovins et caprins).

Enfin, la préparation de l'invention peut être utilisée pour la fabrication d'une composition destinée à limiter le dévéloppement des clostridies chez les lapins atteints d'entérocolite.

L'invention et les avantages qui en découlent ressortiront mieux des exemples de réalisation ci-après.

### EXEMPLE 1

Dans cet exemple, on étudie l'activité inhibitrice d'une huile essentielle de cannelle vis-à-vis du germe Clostridium perfringens isolé du tube digestif de poule.

### Préparation de l'inoculum

On réalise un piquage sur GTS (Gélose Tryptone Soja) d'une culture de Clostridium perfringens isolée du tube digestif de poule. La culture est identifiée sous le numéro CIP99344. On incube 24 heures à 37° C en anaérobiose. On prélève ensuite sur le milieu gélosé une ou plusieurs colonies pour préparer une suspension dans un tube à essai stérile contenant du STS (Solution Tryptone Sel). On mélange ensuite soigneusement au Vortex et on ajuste l'absorbance à 620 nm ± 20 nm avec le STS et ce, afin d'obtenir une suspension contenant environ 10⁸ cellules/ml. A partir de cette suspension, on effectue une série de dilutions jusqu'à 10⁻⁶ avec le STS et ce, pour obtenir une suspension finale contenant environ 10² cellules/ml.

### Préparation de la solution mère (huile essentielle de cannelle)

On prépare une solution mère correspondant à dix fois la plus forte concentration à tester. A partir de cette solution mère, on réalise une gamme de dilution dans des tubes de bouillon Thioglycolate pour que les concentrations correspondent à dix fois les concentrations à tester.

### Préparation du témoin croissance

On prépare ensuite un témoin croissance comme suit. Dans un tube contenant 8 ml de Thioglycolate, on ajoute :
- 1 ml de Thioglycolate
- et 1 ml de la suspension bactérienne contenant environ 10⁵ cellules/ml.

On agite au Vortex. On incube pendant 24 à 48 heures à une température comprise entre 30 et 35° C. On visualise ensuite le trouble du milieu au bout du temps d'incubation.

### Préparation essai

Parallèlement, on prépare l'essai comme suit. Dans 5 tubes contenant 8 ml de Thioglycolate, on ajoute :
- 1 ml de la solution mère à la concentration à tester,
- 1 ml de la suspension bactérienne contenant environ 10⁵ cellules/ml.
On agite au Vortex. On incube pendant 24 à 48 heures à une température comprise entre 30 et 35° C. On visualise le trouble ou non du milieu au bout du temps d'incubation. On réalise un dénombrement sur milieu GTS sur les tubes limites présentant ou non un trouble.

Les résultats figurent dans le tableau ci-après.

| Concentration % | 0,11 | 0,055 | 0,0367 | 0,0275 | 0,022 | 0,0183 |
|---|---|---|---|---|---|---|
| Trouble Témoin | + | + | + | + | + | + |
| Trouble Essai | - | - | - | - | + | + |
| Dénombrement | < 5 | < 5 | < 5 | < 5 | / | / |

Comme le montre ce tableau, on observe une activité inhibitrice de l'huile essentielle de cannelle vis-à-vis de la souche Clostridium perfringens CIP99344 à une concentration 0,0275 %.

### EXEMPLE 2

Dans cet exemple, on étude l'activité inhibitrice de l'huile essentielle de cannelle vis-à-vis du germe Brachyspira hyodysenteriae.

### Matériel et méthode

### A/La souche

La souche de Brachyspira isolée du tube digestif de le porc est repiquée en milieu de WILKINS WEST. Avant chaque essai, la pureté de la souche est vérifiée par sub-culture en gélose Brucella au sang (DIFCO). Enfin une coloration de Gram est systématiquement réalisée.

### B/ Détermination et mesure de la CMI

La CMI est mesurée par la technique de dilution en milieu gélosé selon la norme M II T (1979) et en fonction des recommandations ultérieures de la norme M11 A2 (1990), M11 A3 (1993), M11 A4 (1997) et M11 A5 du NCCLS (2001).

### C/ Préparation de la solution mère et de ses dilutions

Une hydrodispersion d'huile essentielle de cannelle est réalisée extemporanément avec de la saponine à raison de 2 % en poids d'huile essentielle. A partir de ces solutions, une solution-mère (0,05 %) est préparée. Les dilutions successives au demi sont réalisées en eau distillée stérile à partir de cette solution-mère et selon les directives de Ericsson et Sherris.

### D/ Préparation des boîtes de PETRI

Chacune des dilutions du produit est ensuite incorporée en milieu Brucella (DIFCO) additionné de 5 % de sang de cheval. Les gammes de dilution suivantes ont été réalisées de 0,05 % à 0,006 %. Les boîtes ainsi préparées sont séchées 30 minutes avant ensemencement.

### E/ Préparation de l'inoculum

Une pré-culture en milieu de Wilkins West est diluée en bouillon Brucella de façon à obtenir une opacité voisine de l'échelle 0,5 de McFarland. L'inoculum contient alors 10⁷ à 10⁸ UFC/ml.

### F/ Ensemencement des boîtes de PETRI

A l'aide d'un ensemenceur à têtes multiples de type Steers, on dépose sur les boîtes de Petri 2 à 3 µl de l'inoculum préparé et préalablement déposé dans chacune des cupules de l'ensemenceur, de façon à obtenir un inoculum final de 10⁵ UFC par spot d'inoculation.

### G/ Lecture des résultats

Elle est réalisée après 48 heures d'incubation à 37°C en jarre avec sachet générateur de CO₂ (Generbag BioMérieux). La CMI est exprimée en pourcentage.

### Résultats

### 1/ Mesure de l'activité de la saponine

Un témoin de culture en milieu Brucella au sang incubé sous CO₂ est réalisé. Une culture identique est obtenue pour la souche testée (Brachyspira) en présence de saponine.

| Concentration | 20 % | 10 % | 5 % | 2,5 % |
|---|---|---|---|---|
| Culture | Oui | Oui | Oui | Oui |

Comme le montre le tableau ci-dessus, la saponine n'a pas d'activité antibactérienne à la concentration de 20 %, soit dix fois supérieure à celle utilisée dans l'hydrodispersion d'huile.

### 2/ CMI de l'huile essentielle de cannelle

On observe une activité inhibitrice de l'huile essentielle de cannelle vis-à-vis de la souche Brachyspira hyodysenteriae à une concentration égale à 0,006 %.

### EXEMPLE 3

### A/Fabrication de la préparation de l'invention sous forme d'une émulsion

On prépare la composition suivante :

| | |
|---|---|
| Huile essentielle de cannelle | 14 % |
| Tween | 80 % |
| Eau | QSP |

Pour former une émulsion, on soumet les trois constituants à une agitation violente.

### B/Fabrication de la préparation de l'invention sous forme d'une poudre

On prépare un prémélange d'huile essentielle de cannelle et de silice dans un rapport 1/3. On dilue ensuite le produit obtenu sur remoulage à hauteur de 6 %.

## Revendications

1. Utilisation d'une préparation contenant du cinnamaldéhyde pour la fabrication d'une composition destinée au traitement préventif ou curatif des pathologies animales au cours desquelles une présence surnuméraire de germes anaérobie est observée.

2. Utilisation selon la revendication 1, **caractérisée en ce que** le germe appartient au genre Clostridium.

3. Utilisation selon la revendication 2, **caractérisée en ce que** le germe est le Clostridium perfringens.

4. Utilisation selon la revendication 1, **caractérisée en ce que** le germe appartient au genre Brachyspira.

5. Utilisation selon la revendication 4, **caractérisée en ce que** le germe est le Brachyspira hyodysenteriae.

6. Utilisation selon la revendication 1, **caractérisée en ce que** la source de cinnamaldéhyde est un extrait de cannelle.

7. Utilisation selon la revendication 6, **caractérisée en ce que** l'extrait se présente sous la forme d'une huile essentielle.

8. Utilisation selon la revendication 7, **caractérisée en ce que** l'huile essentielle contient entre 50 et 90 % en poids de cinnamaldéhyde, avantageusement entre 70 et 85 % en poids.

9. Utilisation selon la revendication 7, **caractérisée en ce que** la préparation se présente sous la forme d'une émulsion stabilisée dans laquelle l'huile essentielle est dispersée dans une phase aqueuse, en présence d'un agent dispersant.

10. Utilisation selon la revendication 7, **caractérisée en ce que** la préparation se présente sous la forme d'une poudre sur laquelle est adsorbée l'huile essentielle.

11. Utilisation selon la revendication 1, **caractérisée en ce que** la préparation se présente sous forme d'un aliment.

12. Utilisation selon la revendication 1, **caractérisée en ce que** la préparation se présente sous forme d'une eau de boisson.

13. Utilisation selon la revendication 12, **caractérisée en ce que** la boisson contient de 0.1 à 3 ml, avantageusement de 0.35 à 1 ml par litre d'eau de boisson de la préparation.

14. Utilisation selon la revendication 1, **caractérisée en ce que** les pathologies comprennent les dysbactérioses, les entérites non spécifiques, les entérites nécrotiques, et les entérites frilositées se développant chez les volailles.

15. Utilisation selon la revendication 1, **caractérisée en ce que** la quantité de cinnamaldéhyde administrée, dans le cadre d'un traitement préventif, est comprise entre 0,5 x 10⁻³ et 50 x 10⁻³ mg/kg.

16. Utilisation selon la revendication 1, **caractérisée en ce que** la quantité de cinnamaldéhyde administrée, dans le cadre d'un traitement préventif, est comprise entre 3 x 10⁻³ et 350 x 10⁻³ mg/kg de poids vif/jour.

17. Utilisation selon la revendications 1, **caractérisée en ce que** la pathologie est l'entérotoxémie du porc et du ruminant (ovins, bovins, caprins).
